Europäisches Patentamt

European Patent Office

Office européen des brevets

(11) Publication number : **0 513 997 A1**

# (12) EUROPEAN PATENT APPLICATION

(21) Application number : **92303207.2**

(22) Date of filing : **10.04.92**

(51) Int. Cl.$^5$ : **A61K 31/47, A61K 31/485, A61K 31/52**

(30) Priority : **12.04.91 HU 119891**

(43) Date of publication of application :
**19.11.92 Bulletin 92/47**

(84) Designated Contracting States :
**AT BE CH DE DK ES FR GB GR IT LI LU NL PT SE**

(71) Applicant : **CHINOIN Gyogyszer és Vegyészeti Termékek Gyára RT.**
**To utca 1-5**
**H-1045 Budapest IV (HU)**

(72) Inventor : **Kapui, Zoltan**
**58/a Szakasits á. u.**
**H-1115 Budapest (HU)**

Inventor : **Hermecz, István**
**53 Molnar u.**
**H-1056 Budapest (HU)**
Inventor : **Tardos, Lászl**
**34 Egri J. u.**
**H-1111 Budapest (HU)**
Inventor : **Mármarosi, Katalin**
**19 Ybl M. st.**
**H-2051 Biatorbágy (HU)**
Inventor : **Vasvári, Lelle**
**33 Goldmark K. u.**
**H-1122 Budapest (HU)**
Inventor : **Horváth, ágnes**
**5 Regényes u.**
**H-1221 Budapest (HU)**

(74) Representative : **Skailes, Humphrey John et al**
**Frank B. Dehn & Co. Imperial House 15-19 Kingsway**
**London WC2B 6UZ (GB)**

(54) **Antithrombotic compositions.**

(57)   The invention relates to the use of 1-(3',4'-dimethoxy-benzyl)-6,7-dimethoxy-isoquinoline or 1-(3',4'-diethoxy-benzyl)-6,7-diethoxy-3,4-dihydro-isoquinoline or(3',4'-diethoxybenzyl)-6,7-diethoxy-3,4-dihydroisoquinolinium theophyllin-7-acetate or its monohydrate as active ingredient for the manufacture of medicaments for use in the treatment or prevention of conditions resulting from increased thrombocyte - aggregatory activity as a consequence of decreased prostacyclin release.

EP 0 513 997 A1

This invention relates to a pharmaceutical composition containing 1-(3',4'-dimethoxy-benzyl)-6,7-dimethoxy-isoquinoline (further on Papaverine) or 1-(3-,4'-diethoxybenzyl)-6,7-diethoxy-3,4-dihydro-iso-quinoline (further on Nospa) or (3',4'-diethoxy-benzyl)-6,7-diethoxy-3,4-dihydroisoquinolinium theophyllin-7-acetate or its monohydrate (further on Depogen) as active ingredient, and processs for the preparation thereof. The pharmaceutical composition according to the present invention is prepared in a way that

Papaverine or

Nospa or

Depogen or

its monohydrate, produced by methods known per se, is mixed with filling- diluting- and auxiliary materials commonly used in drug manufactoring and is converted into a pharmaceutical preparation useful in the treatment or prevention of conditions resulting from increased thrombocyte aggregation activity as a consequence of decreased prostacycline release.

Papaverine may be produced by methods described in the literature among others in Compt. Rend. 149, 210 (1909), Ber. 42, 29-45 (1909) and in Hungarian Patent Specifications 108.865, 149.740, 155.539 and 171.731. Nospa may be produced as described in Hungarian Patent Specification 150.535, and Depogen as described in Hungarian Patent Specifications 167.246 and 194.179.

It is known from US-SP 3.035.366 and from Acta Pharmaceutica Hungarica 49, 50-53 (1979) that Depogen in a dose of 100-300 mg/day has a peripheral vasodilating effect and decreases the extremital vascular resistance. Depogen improves some rheological parameters of the blood, particulary the plasticity of the erythrocytes and thus it can be used for treating clinical conditions with good results where poor microcirculation and secondary tissue hypoxia is a consequence of the deteriation of the plasticity of the erythrocytes.

By influencing the haemorheological properties of the blood, namely increasing its fluidity, many pathological conditions may favourably be influenced (e.g. decrease of the danger of thrombosis after operations, prophylaxis of myocardial infarcts, slowly healing wounds caused by poor blood supply, ulcers, embolism, cerebral infarcts, shock, chronic vascular diseases, claudication, senile cerebral circulatory disturbancess, diabetes) and this is of great therapeutical importance both systemically and locally (Hungarian Patent Specification 197.207). According to Hungariam Patent Specification 197.207 Depogen by improving the haemorheological parameters of the blood favourably influences the thrombocyte functions, e.g. the aggregation.

We found that the active ingredients of the compositions according to the present invention exert their effect on the platelets not only indirectly by improving the haemorheological parameters of the blood, but also directly.

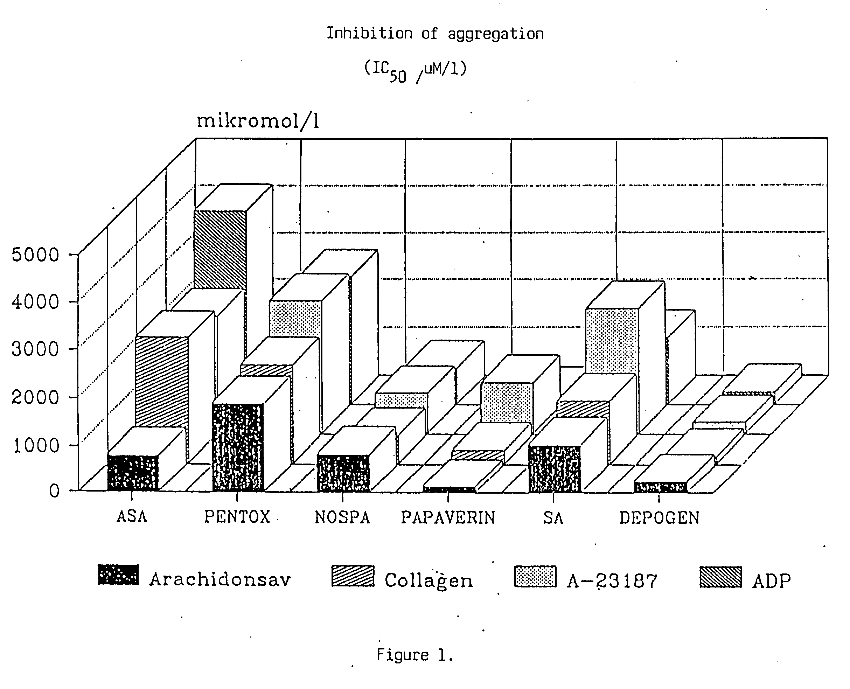

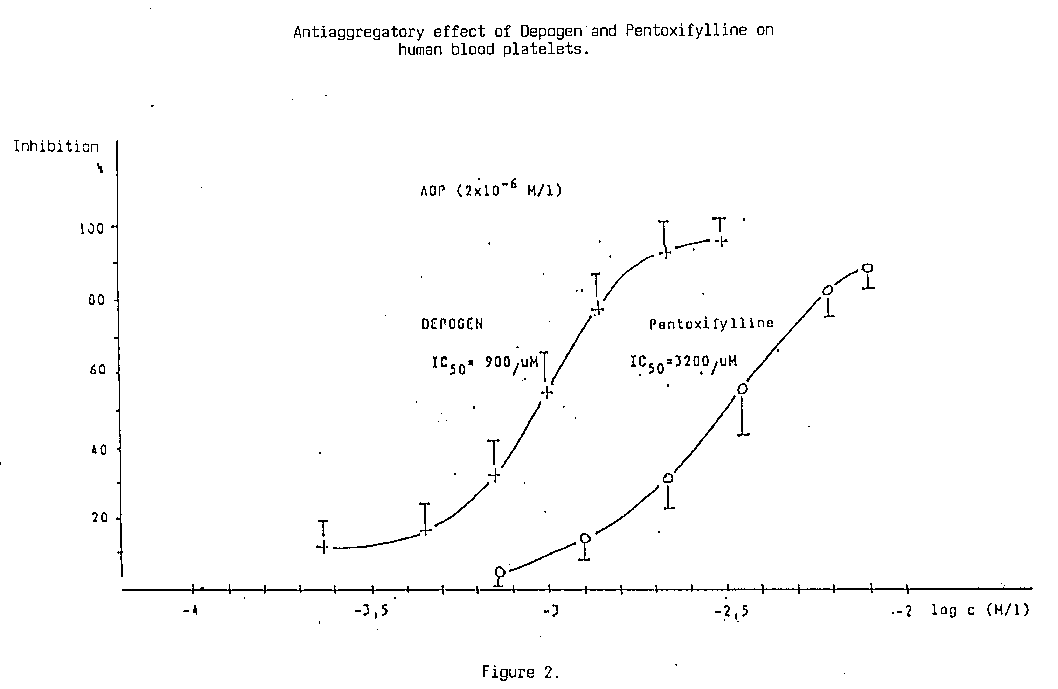

We compared on rabbit platelets the in vitro antiaggregatory effect , of acetylsalicylic acid (ASA), salicylic acid (SA). Pentoxifylline (Pentox or Trental), Nospa, Papaverine and Depogen. The aggregation of the rabbit platelets was induced by adenosine diphosphate (ADP), arachidonic acid (AA), Collagen and A 23187 Ca ionophor. The investigated compounds inhibited the platelet aggregation in vitro, and among them Depogen had the strongest effect (Figure 1, 2 and Table 1).

## Table 1.

### In vitro $ID_{50}$ values of the investigated compounds on rabbit platelet aggregation.

| | ADP | Collagen | AA | A-23187 |
|---|---|---|---|---|
| | | $IC_{50}$ $(\mu M)$ | | |
| ASA | 4050 | 2610 | 780 | 2440 |
| PENTOX | 2660 | 2050 | 1870 | 2770 |
| Nospa | 740 | 580 | 810 | 840 |
| Papaverine | 190 | 270 | 130 | 1060 |
| Salicylic acid | 1380 | 1300 | 1010 | 2610 |
| Depogen | 247 | 140 | 240 | 230 |

Both the figures and the table indicate that the antiaggregatory effect of Depogen is 10-20 times stronger than that of the worldwide used antiaggregatory agent, ASA.

According to our latest experiments, Depogen not only inhibits the platelet aggregation induced by arachidonic acid, ADP, collagen and A 23187 Ca ionophor, but also antagonizes the platelet aggregation induced by the Platelet Activating Factor (PAF).

Depogen inhibits in dose dependent manner the by 1 μM of PAF induced platelet aggregation.

Table 2

| Compound | Inhibition of PAF induced platelet aggregation IC50 (μM/l) |
|---|---|
| Papaverin | 656 |
| Depogen | 684 |
| Nospa | 756 |
| Pentoxifylline | 1000 |

Simultaneously with the inhibition of the PAF-induced aggregation, Depogen inhibits the by $5 \times 10^{-8}$ M of PAF induced serotonine release in the rabbit platelets.

Table 3

| Compound | Inhibition of Serotonine release IC50 (μM/l) |
|---|---|
| Papaverine | 48.2 |
| Depogen | 118 |
| Nospa | 96 |
| Pentoxifylline | 800 |

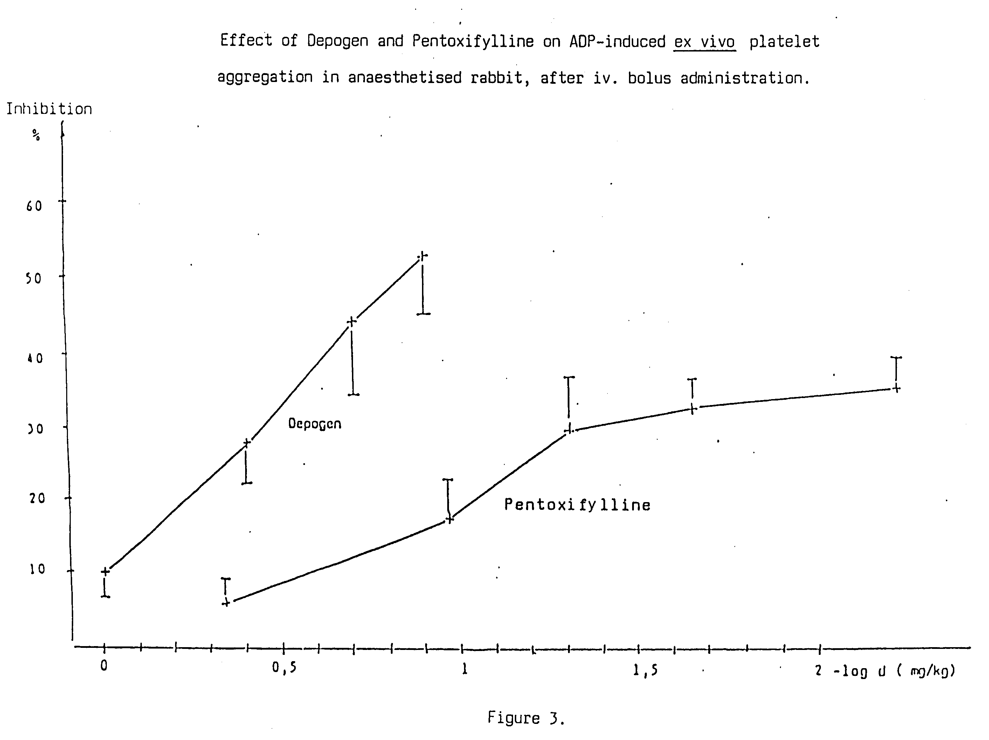

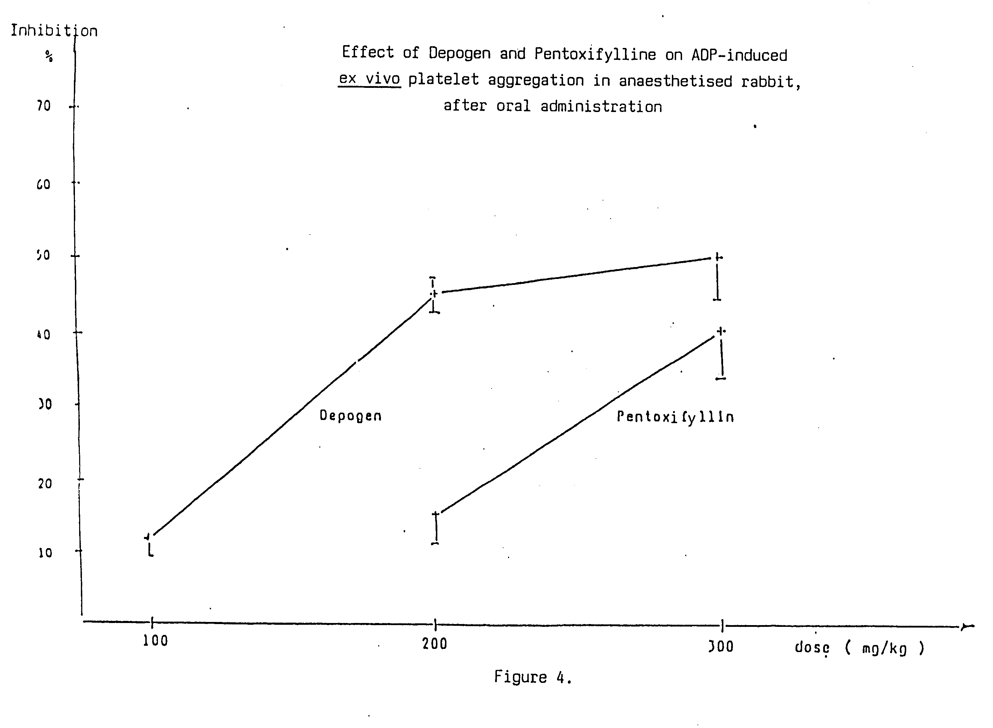

Depogen exerts significant antiaggregatory effect also in ex vivo experiments, both after oral and intravenous administration (Figures 3 and 4). This effect of Depogen is much stronger than that of Pentoxifylline.

We investigated furthermore the in vivo antithrombotic effect of Depogen on anaesthetised rabbits. Depogen was administered intravenously in a dose range of 2.5-7.5 mg/kg. The vena yugularis was connected with the arteria carotis with the help of a plastic vessel. In the plastic vessel a cotton thread was placed, which served as inductor. The amount of thrombus sticked to the surface of the cotton was weighed in the case of the treated and untreated animals (after Herrmann R.G. et al., Proc. Soc. Exp. Biol. Med. 139, 548-552, 1972.)

Table 4

| Depogen dose (mg/kg) | Inhibitory Effect % | |
|---|---|---|
| | ex vivo aggregation | in vivo thrombus formation |
| 2.5 | 30± 4 | 25± 3 |
| 5.0 | 45± 5 | 45± 5 |
| 7.5 | 55± 6 | 60± 7 |

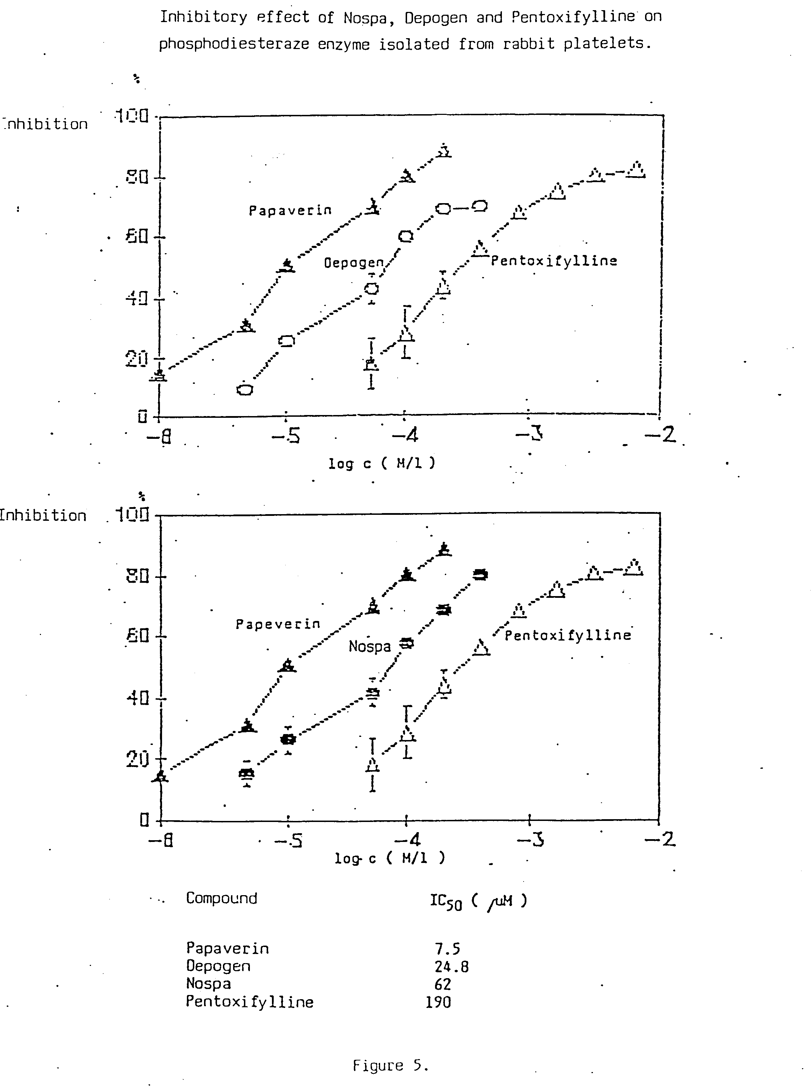

Of the active ingredients according to the present invention the mechanism of the antiaggregatory effect of Depogen was investigated in detail. We proved by in vitro experiments that it is the strong phosphodiesterase inhibitory effect of Depogen which constitutes the basis of its antiaggregatory effect (Figure 5, Table 5).

Table 5

| Compound | PDE Inhibition | | Antiaggregatory Effect | | |
|---|---|---|---|---|---|
| | | AA | Collagen | A 23187 | ADP |
| Papaverin | 7.5 | 130 | 270 | 1060 | 190 |
| Depogen | 24.8 | 240 | 140 | 228 | 247 |
| Nospa | 62 | 810 | 580 | 840 | 740 |
| Pentoxifylline | 190 | 1870 | 2050 | 2770 | 2660 |

Depogen, owing to its phosphodiesterase inhibitory effect increases the intracellular cAMP (cyclic-adenosine-monophosphate) level of the platelets. The increased cAMP level inhibits the cyclooxygenase enzyme, which produces the strongly aggregatory thromboxane $A_2$. On the other hand the increased cAMP level inhibits the phospholipase Az enzyme in the platelets, which catalyses the liberation of the arachidonic acid - the substrate of the cyclooxygenase enzyme - and by this way too, inhibits the thromboxane $A_2$ production.

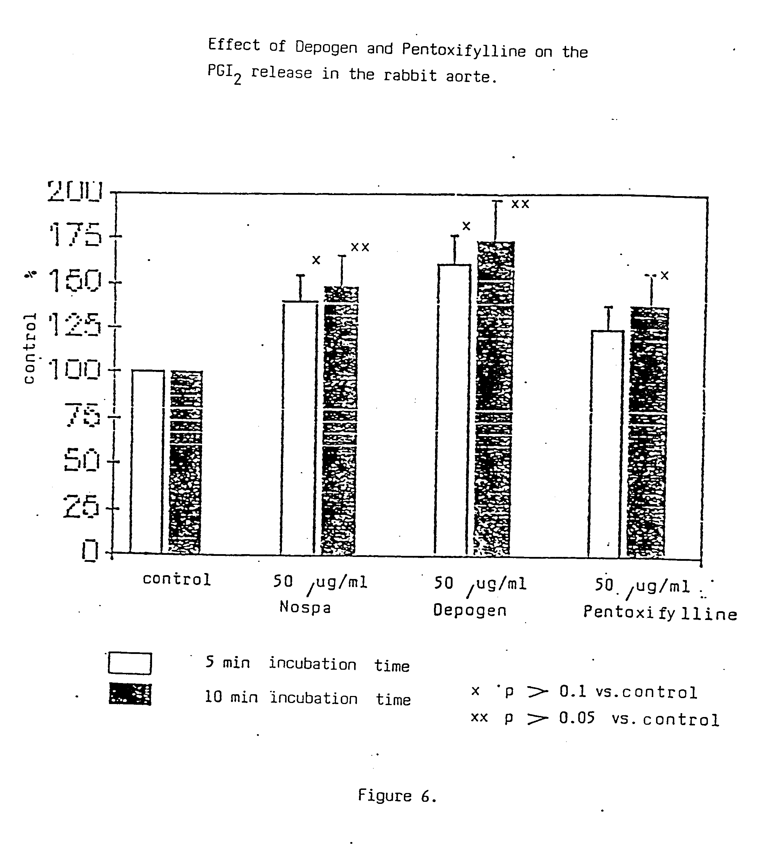

As shown by in vitro experiments, Depogen significantly enhances the prostacyclin release from the aorta (Figure 6). From the point of view of $PGI_2$ release Depogen represents a considerable adventage compared to Pentoxifylline, as the ratio of the doses inhibiting platelet aggregation and increasing $PGI_2$ release is lower for Depogen than for Pentoxifylline (Table 6) and thus the effective dose ranges of the two actions are closer to each other in the case of Depogen.

Table 6

Aggregation induced by

| Compound | AA | Collagen | A 23187 | ADP |
|---|---|---|---|---|
| | Dose inhibiting aggregation | | | |
| | Dose inreasing PGI2 release | | | |
| Depogen | 3.3 | 1.8 | 3.14 | 3.4 |
| Pentoxifylline | 10.5 | 11.5 | 15.5 | 14.9 |

We investigated the effect of Depogen on the thromboxane and prostacyclin level of the blood plasma, in rat, after 4 days of per os administration in doses of 5 mg/kg and 10 mg/kg. On the 5th day we determined the thromboxane and prostacyclin level of the blood plasma (Table 7).

Table 7

| Depogen p.o. dose mg/kg/day during 4 days | TxA2 | PGI2 pg/ml of plasma | PGI2/TxA2 |
|---|---|---|---|
| Control | 59.2± 2.9 | 227.7± 12.3 | 3.8 |
| 5 mg/kg | 48.7± 3.9 | 206.4± 11.2 | 4.2 |
| 10 mg/kg | 33.7± 3.6 | 363.8± 19.3 | 11 |

10 mg/kg Depogen decreased significantly the thromboxane level and increased the $PGI_2$ level in the plasma, and shifted the $PGI_2$ - thromboxane balance toward the $PGI_2$. This shift of the $PGI_2$ - thromboxane balance towards the $PGI_2$ is a fundamental point of the antithrombotic action of Depogen.

The above data demonstrate that the active ingredients of the pharmaceutical composition according to the present invention are new-type antithrombotic agents. They exert their activity in two different ways. On one hand they directly inhibit platelet aggregation, on the other hand they indirectly decrease the aggreability of the platelets by increasing the plasma $PGI_2$ level.

No antithrombotic agent with similar double mechanism of action is known in the literature.

The active ingredients according to the present invention, apart from their known therapeutic fields, may also be used in pathological cases where a salicylic acid derivative e.g. Colfarit is used with succes, to substitute the salicylic acid derivative.

The active ingredients according to the present invention, first of all Depogen, have further advantages compared with the widespreadly used ASA. ASA has significant gastrointestinal adverse effects, such as peptic ulcer, gastric haemorrhage. Depogen has no such adverse effect, and what is more, it has a weak ulcus-preventing activity. According to the measurements, in the antiaggregatory dose it exerts a 25-50 % inhibition on the acified ethanol- and indomethacine- induced ulceration in rat.

In the treatment the drug may be used in form of capsules, tablets, retard tablets, injections, solutions or syrups in bolus or infusion.

The usual dose depending from the age, weight and severity of the illness may vary between 100-400 mg/day.

The active ingredients of the pharmaceutical compositions according to the present invention are produced by methods known per se. The expression " known per se" includes all the processes known in the art before the priority date of the present application. By processes known in the drug production tablets and injections of the following compositions are prepared:

Example 1.    Tablet 80 mg.  (1)

| 1-(3´,4´-Diethoxybenzyl)-6,7-diethoxy-3,4-dihydroisoquinolinium hydrochloride | 80 mg |
|---|---|
| Magnesium stearate | 6 mg |
| Talcum | 8 mg |
| Polyvidon | 12 mg |
| Starch | 70 mg |
| Lactose | 104 mg |

Example 2.    Tablet 80 mg.  (2)

| 1-(3´,4´-Dimethoxybenzyl)-6,7-dimethoxy-3,4-dihydroisoquinolinium hydrochloride | 80 mg |
|---|---|
| Magnesium stearate | 4 mg |
| Talcum | 6 mg |
| Gelatin alba | 8 mg |
| Amylum-zolami | 62 mg |
| Lactose | 120 mg |

Example 3.    Tablet 160 mg.

| 1-(3´,4´-Diethoxybenzyl)-6,7-diethoxy-3,4-dihydroisoquinolinium 1,3-dimethyl-xanthin-yl-acetate | 150 mg |
|---|---|
| Magnesium stearate | 12 mg |
| Talcum | 15 mg |
| Polyvidon | 20 mg |
| Starch | 130 mg |
| Lactose | 205 mg |

Example 4.    Tablet 160 mg.

1-(3´,4´-Diethoxybenzyl)-6,7-diethoxy-3,4-
dihydroisoquinolinium 1,3-dimethyl-xanthin-yl-
acetate-monohydrate                                    160 mg

Magnesium stearate                                      14 mg

Talcum                                                  18 mg

Polyvidon                                               20 mg

Starch                                                 135 mg

Lactose                                                210 mg


Example 5.    Tablet 40 mg.   (1)

1-(3´,4´-Dimethoxybenzyl)-6,7-dimethoxy-3,4-
dihydroisoquinolinium hydrochloride                     40 mg

Magnesium stearate                                       2 mg

Talcum                                                   3 mg

Gelatin alba                                             4 mg

Amylum-solan                                            31 mg

Lactose                                                120 mg


Example 6.    Tablet 40 mg.   (2)

1-(3´,4´-Diethoxybenzyl)-6,7-diethoxy-3,4-
dihydroisoquinolinium hydrochloride                     40 mg

Magnesium stearate                                       3 mg

Talcum                                                   4 mg

Polyvidon                                                6 mg

Starch                                                  35 mg

Lactose                                                 52 mg

7

Example 7.   Injection   40 mg.   (1)

| | |
|---|---|
| 1-(3´,4´-Diethoxybenzyl)-6,7-diethoxy-3,4-dihydroisoquinolinium hydrochloride | 40 mg |
| Sodium pyrosulphurosum | 2 mg |
| Ethanol 96 % v/w | 132 mg |
| Water | 2 ml |

Example 8.   Injection   40 mg.   (2)

| | |
|---|---|
| 1-(3´,4´-Dimethoxybenzyl)-6,7-diethoxy-3,4-dihydroisoquinolinium hydrochloride | 40 mg |
| Sodium pyrosulphurosum | 2 mg |
| Ethanol 96 % v/w | 130 mg |
| Water | 2 ml |

Example 9.   Tablet 100 mg.

| | |
|---|---|
| 1-(3´,4´-Diethoxybenzyl)-6,7-diethoxy-3,4-dihydroisoquinolinium hydrochloride | 100 mg |
| Magnesium stearate | 8 mg |
| Talcum | 10 mg |
| Polyvidon | 15 mg |
| Starch | 82 mg |
| Lactose | 120 mg |

**Claims**

1.  The use of 1-(3-,4-dimethoxy-benzyl)-6,7-dimethoxy-isoquinoline or 1-(3′,4′-diethoxy- benzyl)-6,7-diethoxy-3,4-dihydro-isoquinoline or (3′,4′-diethoxybenzyl)-6,7-diethoxy-3,4-dihydroisoquinolinium theophyllin-7-acetate or its monohydrate as active ingredient for the manufacture of medicaments for use in the treatment or prevention of conditions resulting from increased thrombocyte - aggregatory activity as a consequence of decreased prostacyclin release.

2.  Pharmaceutical composition according to Claim 1, **which comprises** presenting the drug in the form of capsules, tablets, retard tablets, injections, solutions of syrups.

3.  Process for the preparation of a pharmaceutical composition according to Claim 1, **which comprises** mixing 1-(3′,4′-dimethoxy-benzyl)-6,7-dimethoxy-isoquinoline or 1-(3′,4′-diethoxy benzyl)-6,7-diethoxy-3,4-dihydro-isoquinoline or (3′,4′-diethoxy-benzyl)-6,7-diethoxy-3,4-dihydroisoquinolinium theophyllin-7-acetate or its monohydrate produced by methods known per se with filling- diluting- and auxiliary materials commonly used in the drug manufactoring, and converting them into pharmaceutical preparations useful in the treatment or prevention of conditions resulting from increased thrombocyte-aggregatory activity as a consequence of decreased prostacyclin-release.

4. A method of treating or prevention of conditions resulting from increased thrombocyte aggregatory activity as a consequence of decreased prostacyclin release in an animal subject, **which comprises** the step of administering 1-(3′,4′-dimethoxy-benzyl)-6,7-dimethoxy-isoquinoline or 1-(3′,4′-diethoxy-benzyl)-6,7-diethoxy-3,4-dihydro-isoquinoline or (3′,4′-diethoxy-benzyl)-6,7-diethoxy-3,4-dihydroisoquinolinium theophyllin-7-acetate or its monohydrate in an effective dosage.

Inhibition of aggregation

$(IC_{50} / \text{uM}/1)$

mikromol/l

ASA    PENTOX    NOSPA    PAPAVERIN    SA    DEPOGEN

▓ Arachidonsav    ▨ Collagen    ▨ A-23187    ▨ ADP

Figure 1.

EP 0 513 997 A1

Antiaggregatory effect of Depogen and Pentoxifylline on human blood platelets.

Inhibition %

Pentoxifylline
$IC_{50} = 3200 \, \mu M$

ADP $(2 \times 10^{-6} \, M/1)$

DEPOGEN
$IC_{50} = 900 \, \mu M$

log c (M/1)

Figure 2.

11

Effect of Depogen and Pentoxifylline on ADP-induced <u>ex vivo</u> platelet

aggregation in anaesthetised rabbit, after iv. bolus administration.

Figure 3.

EP 0 513 997 A1

EP 0 513 997 A1

Effect of Depogen and Pentoxifylline on ADP-induced
ex vivo platelet aggregation in anaesthetised rabbit,
after oral administration

Inhibition %

70

60

50

40

30

20

10

Depogen

Pentoxifyllin

100

200

300

dose ( mg/kg )

Figure 4.

13

Inhibitory effect of Nospa, Depogen and Pentoxifylline on
phosphodiesteraze enzyme isolated from rabbit platelets.

| Compound | IC_50 ( µM ) |
| --- | --- |
| Papaverin | 7.5 |
| Depogen | 24.8 |
| Nospa | 62 |
| Pentoxifylline | 190 |

Figure 5.

Effect of Depogen and Pentoxifylline on the
$PGI_2$ release in the rabbit aorte.

Figure 6.

European Patent
Office

**EUROPEAN SEARCH REPORT**

Application Number

EP    92 30 3207

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 227 356 (CHINOIN GYOGYSZER ES VEGYESZETI TERMEKEK GYARA RT) <br> * page 2, line 27 - line 30 * <br> --- | 1-4 | A61K31/47 <br> A61K31/485 <br> A61K31/52 |
| A | CHEMICAL ABSTRACTS, vol. 83, no. 13, 29 September 1975, Columbus, Ohio, US; abstract no. 108335R, V.F. KREMNEVA ET AL.: 'Effect of dibazole, euphylline, No-Spa and halidor on the osmotic resistance of thrombocytes and on blood clot retraction' page 36 ; & FARMAKOL. TOKSIKOL. vol. 38, no. 3, 1975, pages 324 - 327; * abstract * <br> --- | 1-4 | |
| X | ACTA MED. ACAD. SCI. HUNG. vol. 31, no. 3-4, 1975, pages 157 - 163; H. LOSONCZY ET AL.: 'Effect of prostaglandins and drotaverine on ADP-induced platelet aggregation' * page 160, last paragraph * <br> --- | 1-4 | TECHNICAL FIELDS SEARCHED (Int. Cl.5) <br><br> A61K |
| X | FOLIA HAEMATOL. vol. 92, no. 4, 1969, pages 470 - 476; R. FARBISZEWSKI ET AL.: 'Einfluss von verschiedenen Arzneimitteln auf die Thrombocytenaggregation und die Freisetzung des Blutplättchenfaktors 4 (Antiheparinfaktor)' * page 474, paragraph 1-2; table I * <br> --- <br> -/-- | 1-4 | |

The present search report has been drawn up for all claims

| Place of search | Date of completion of the search | Examiner |
|---|---|---|
| MUNICH | 23 JULY 1992 | FOERSTER W.K. |

EP 0 513 997 A1

| | **European Patent Office** | **EUROPEAN SEARCH REPORT** | Application Number |

EP 92 30 3207
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROC. NATL. ACAD. SCI. USA vol. 83, 1986, pages 6660 - 6663; C.H. MACPHEE: 'Immunological identification of the major platelet low km cAMP phosphodiesterase: PRobable target for anti.thrombotic agents' * page 6660, left column, last paragraph * * page 6663, left column, paragraph 1; table I * | 1-4 | |
| X | AGENTS AND ACTIONS vol. 17, no. 2, 1985, pages 220 - 228; F. DE CLERCK ET AL.: 'Biochemical mechanisms in 5-hydroxytryptamine-induced human platelet aggregation' * page 226, left column, paragraph 3 - right column, line 10 * | 1-4 | |
| | | | TECHNICAL FIELDS SEARCHED (Int. Cl.5) |

The present search report has been drawn up for all claims

| Place of search | Date of completion of the search | Examiner |
|---|---|---|
| MUNICH | 23 JULY 1992 | FOERSTER W. K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)